# FASCICULE DE BREVET EUROPEEN

(11) **EP 3 643 229 B1**
(45) Date de publication et mention de la délivrance du brevet: **15.03.2023**
(21) Numéro de dépôt: 19199096.9
(22) Date de dépôt: 24.09.2019
(51) Int. Cl.: A61B 5/08, A61B 5/083, A61B 5/0205, A61B 5/318, A61B 5/361

(54) **APPAREIL DE MONITORAGE CARDIAQUE POUR RÉANIMATION CARDIO-PULMONAIRE AVEC AFFICHAGE DES TENEURS MAXIMALE OU MOYENNE EN CO2**
KARDIALES ÜBERWACHUNGSGERÄT FÜR DIE HERZ-LUNGEN-REANIMATION MIT ANZEIGE DER MAXIMALEN ODER MITTLEREN CO2-WERTE
CARDIAC MONITORING APPARATUS FOR CARDIOPULMONARY RESUSCITATION WITH DISPLAY OF MAXIMUM OR AVERAGE CO2 CONTENT

(30) Priorité: 23.10.2018 FR 1859783
(43) Date de publication de la demande: 29.04.2020
(73) Titulaire: Air Liquide Medical Systems, 92160 Antony Cedex (FR)
(72) Inventeur: RICHARD, Jean-Christophe, 92160 ANTONY (FR); RIGOLLOT, Marceau, 92160 ANTONY (FR); BADAT, Bilal, 92160 ANTONY (FR)
(74) Mandataire: Air Liquide

(56) Documents cités:
- WO-A1-2012/114286
- US-A1- 2018 125 375
- US-A1- 2018 177 678
- US-A1- 2018 235 510

## Description

L'invention porte sur un appareil médical de monitorage cardiaque permettant de suivre un ou des signes ou paramètres vitaux, en particulier l'activité électrique du coeur et la teneur en CO₂ dans les gaz expirés, chez un patient en arrêt cardio-respiratoire, en particulier avant, pendant et/ou après une réanimation cardio-pulmonaire, c'est-à-dire un massage cardiaque par un secouriste.

Lors de la prise en charge d'une personne, i.e. un patient, en arrêt cardio-pulmonaire ou ACR, les secouristes relient généralement le patient à un appareil de monitorage cardiaque, aussi appelé moniteur cardiaque, de signes vitaux du patient, en particulier de l'activité électrique du coeur de ce patient. Il peut être associé ou non à un défibrillateur cardiaque.

Un appareil de monitorage cardiaque peut mesurer et afficher des mesures d'un ou plusieurs paramètres physiologiques ou signes vitaux du patient, notamment cardiaques, par exemple :
- l'activité électrique cardiaque, i.e. électrocardiogramme (ECG), et/ou la fréquence cardiaque du patient, qui sont mesurées par des électrodes placées sur le patient,
- la saturation en oxygène (SpO₂) du patient, mesurée via un capteur transcutané,
- la température via un capteur de température en contact avec le corps du patient, et/ou
- la pression artérielle (PA) grâce à un brassard de mesure de pression artérielle.

Ces mesures permettent de connaître et suivre l'état de santé du patient, en particulier avant et pendant que les secouristes interviennent sur le patient en ACR, en lui pratiquant une réanimation cardio-pulmonaire (RCP) avec massage cardiaque dans le but de faire repartir le coeur, mais aussi après interruption de la RCP.

Toutefois, suivre ces signes physiologiques vitaux du patient, notamment cardiaques, est insuffisant et il est recommandé de suivre également, i.e. monitorer, pendant la RCP, les composés gazeux présents non seulement dans le gaz administré au patient mais aussi ceux présents dans les gaz expirés par le patient, en particulier la quantité de CO₂ qui s'y trouve.

Le CO₂ est produit par le métabolisme du patient, transporté jusqu'aux poumons grâce à la circulation sanguine et évacués lors de l'expiration du fait des échanges gazeux ayant lieu dans les poumons. Les variations de teneur en CO₂ dans les gaz expirés par un patient au fil du temps (en secondes) sont généralement représentées graphiquement sous forme d'un capnogramme.

Plus précisément, l'etCO₂ pour « End Tidal CO₂ » ou CO₂ en fin d'expiration, correspond à la mesure de la fraction de CO₂ expirée qui est présente dans les gaz recueillis lors de l'expiration du patient.

Pendant une RCP, le CO₂ alvéolaire, qui dépend non seulement des rapports ventilation/perfusion pulmonaire mais aussi de la quantité de CO₂ généré par le métabolisme cellulaire, représente un paramètre très utile au secouriste, par exemple un médecin ou analogue, pour juger de l'efficacité de la RCP. En effet, plus la RCP est efficace, plus le débit cardiaque généré par les compressions thoraciques est important, et donc plus la quantité de CO₂ ramenée vers les poumons est importante.

Le monitorage non-invasif de l'etCO₂, qui reflète indirectement le CO₂ alvéolaire, est donc de plus en plus utilisé pendant les RCP pour renseigner le secouriste pratiquant le massage cardiaque. Un tel monitorage de l'etCO₂ a déjà été proposé par WO-A-2012/114286 ou US-A-2018/0177678.

Différentes techniques permettent l'analyse par spectrophotométrie de la teneur en CO₂ des gaz expirés. Par exemple, le gaz expiré peut être :
- soit convoyé puis analysé par une cellule d'analyse sur un site déporté. Cette manière d'opérer est appelée à flux dérivé ou secondaire, i.e. « *sidestream* » en anglais,
- soit analysé près du patient. Cette manière d'opérer est appelée à flux principal, i.e. « *mainstream* » en anglais.

Les documents WO-A-2014/072981, US-A-2016/133160 et US-A-2012/016279 proposent des méthodes de suivi de la teneur en CO₂ dans les gaz expirés par un patient subissant une RCP, dans lesquelles les ventilateurs médicaux utilisés indiquent au secouriste qu'il doit stopper le massage cardiaque lorsque la teneur en etCO₂ est supérieure à 30 mm Hg par exemple.

Toutefois, ces solutions de monitorage de l'etCO₂ sont adaptées aux variations de CO₂ provoquées par la respiration, qu'elle soit mécanique ou spontanée. Les fréquences enjeu sont donc de l'ordre de 10 à 40 c/min. Les algorithmes et mécanismes mis en oeuvre sont adaptés à ces fréquences et à des variations faibles du CO₂ entre deux respirations du patient.

Or, pendant une RCP, les fréquences des CT enjeu sont proches de 100 c/min, les volumes de gaz mobilisés faibles et les débits de gaz importants et irréguliers. De plus, un problème d'espaces morts s'ajoute à ces difficultés puisque, du fait des CT, une même fraction de gaz peut être analysée plusieurs fois par le capteur de CO₂, à défaut de mise en place d'un rinçage ou purge de l'espace mort de l'appareillage.

Dans ces conditions, la valeur de l'etCO₂ affichée par les appareils de monitorage actuels est rafraîchie à une fréquence inadéquate puisque ceux-ci tentent de suivre l'évolution du CO₂ à la fréquence du massage, à savoir 100 c/min. Il s'ensuit alors que les valeurs d'etCO₂ affichées par les moniteurs actuels ne sont pas représentatives d'une concentration de CO₂ liée au métabolisme du patient et sont dès lors souvent erronées. En d'autres termes, l'etCO₂ tel qu'il est mesuré actuellement, c'est-à-dire lors de chaque CT, ne permet pas d'obtenir une approximation fiable du CO₂ alvéolaire.

Ceci est un problème car le CO₂ alvéolaire est un paramètre vital très important puisque non seulement il reflète la qualité de la RCP mais surtout il est annonciateur d'une possible reprise d'activité cardiaque spontanée ou « RACS ».

En effet, pendant une RCP, la valeur de concentration en CO₂ ou la tendance en CO₂ est utilisée par le secouriste pratiquant le massage cardiaque, i.e. médecin ou tout autre personnel soignant, comme une « image » de la circulation sanguine et donc de l'efficacité du massage cardiaque qu'il pratique, la tendance en CO₂ étant définie comme une représentation graphique de plusieurs valeurs de concentration en CO₂ mesurées successivement sur une fenêtre de temps donnée, par exemple pendant les 30 secondes à 5 minutes venant de s'écouler.

Le problème alors est de proposer un appareil de monitorage cardiaque ou moniteur cardiaque amélioré, lequel permette d'opérer un monitorage ou suivi efficace non seulement d'un ou plusieurs signes ou paramètres vitaux du patient, en particulier son activité électrique cardiaque, mais aussi de la teneur en CO₂ dans les gaz expirés par le patient, pendant la réalisation d'une RCP avec massage cardiaque de sorte de pouvoir suivre les variations de teneur en CO₂ dans les gaz expirés par le patient et préférentiellement d'informer ou d'alerter le personnel soignant en cas de RACS, ledit moniteur cardiaque pouvant être associé ou non à un défibrillateur cardiaque.

La solution de l'invention porte sur un appareil de monitorage cardiaque, c'est-à-dire un moniteur cardiaque de paramètres ou signes vitaux, en particulier l'activité électrique cardiaque et la teneur en CO₂ dans les gaz expirés, permettant de suivre et/ou de monitorer un ou plusieurs paramètres ou signes vitaux d'un patient en arrêt cardio-respiratoire, avant, pendant et/ou après la mise en oeuvre d'un massage cardiaque sur ledit patient, lequel peut éventuellement comprendre des moyens de défibrillation, c'est-à-dire être un défibrillateur.

Plus précisément, l'invention concerne un appareil de monitorage cardiaque d'un patient, c'est-à-dire d'une personne humaine, en particulier d'un patient en arrêt cardio-respiratoire (ACR) pendant une réanimation cardio-pulmonaire (RCP), comprenant :
- des moyens de mesure de l'activité cardiaque du patient,
- au moins une interface graphique utilisateur (IGU),
- des moyens de mesure de teneur en CO₂ pour opérer des mesures de concentration en CO₂ produit par ledit patient et fournir des signaux de mesure de teneur en CO₂ à des moyens de traitement de signal,
- des moyens de traitement de signal configurés pour traiter les signaux de mesure de teneur en CO₂ provenant des moyens de mesure de teneur en CO₂, et
- des moyens de mémorisation pour enregistrer au moins les valeurs de CO₂ traitées,
caractérisé en ce que :
A) les moyens de traitement de signal sont configurés pour :
   i) sélectionner une valeur maximale (Vmax) de teneur en CO₂ parmi une pluralité de valeurs de teneur en CO₂ mesurées pendant une période de temps (dt) donnée, et enregistrées dans les moyens de mémorisation,
   ii) répéter l'étape i) pour obtenir plusieurs valeurs maximales (Vmax) de teneur en CO₂ successives mesurées sur une fenêtre de temps (Ft) comprenant plusieurs périodes de temps (dt) successives,
   iii) calculer au moins une valeur moyenne (Vmoy) de teneur en CO₂ à partir des valeurs maximales (Vmax) de teneur en CO₂ obtenues sur la fenêtre de temps (Ft),
   iv) répéter les étapes i) à iii) de manière à obtenir plusieurs valeurs moyennes (Vmoy) de teneur en CO₂ successives calculées à partir de valeurs maximales (Vmax) de teneur en CO₂ obtenues sur plusieurs fenêtres de temps (Ft) successives, et
   v) transmettre les valeurs moyennes (Vmoy) de teneur en CO₂ successives à l'interface graphique utilisateur (IGU), et
B) l'interface graphique utilisateur est configurée pour afficher les valeurs moyennes (Vmoy) de teneur en CO₂ successives sous forme d'au moins une représentation graphique.

Dans le cadre de l'invention, les valeurs des différentes teneurs en CO₂ peuvent être exprimées en n'importe quelle unité, par exemple pression partielle de CO₂ exprimée en mmHg ou en kPa par exemple, pourcentage molaire ou volumique, ou tout autre grandeur.

Selon le cas, l'appareil de monitorage cardiaque de l'invention peut comprendre l'une ou plusieurs des caractéristiques techniques suivantes :
- la fenêtre de temps (Ft) est une fenêtre de temps glissante.
- l'IGU est configurée pour afficher toutes ou partie des valeurs moyennes (Vmoy) de teneur en CO₂ successives.
- l'interface graphique utilisateur est en outre configurée pour afficher au moins une valeur moyenne (Vmoy) de teneur en CO₂ sous forme d'au moins une valeur numérique.
- l'interface graphique utilisateur est configurée pour afficher au moins une partie des valeurs moyennes (Vmoy) successives de teneur en CO₂ calculées sous forme :
   . d'une courbe formée d'une succession de symboles graphiques, chaque symbole graphique correspondant à une valeur moyenne (Vmoy) de teneur en CO₂, ou
   . d'un barre-graphe comprenant plusieurs barres, chaque barre dudit barre-graphe correspondant à une valeur moyenne (Vmoy) de teneur en CO₂,
   . ou de toute autre type de représentation (i.e. icones, symboles graphiques, etc...).
   - l'IGU est configurée pour afficher une courbe de tendance formée d'une succession de symboles graphiques correspondant chacun à une valeur moyenne (Vmoy) de teneur en CO₂. En d'autres termes, l'IGU affiche sur un graphique temporel représentant une représentation graphique de chaque valeur moyenne (Vmoy) de teneur en CO₂, à savoir un symbole graphique de type point ou croix par exemple, en fonction du temps (en secondes ou minutes). Pour les valeurs moyenne (Vmoy) de teneur en CO₂, l'affichage se fait de préférence sur une fenêtre de temps glissante allant de 30 sec à 5 minutes par exemple, en particulier de 1 à 3 minutes.
   - la période de temps (dt) est comprise entre 2 et 10 secondes, typiquement de l'ordre de 3 à 6 secondes.
   - la fenêtre de temps (Ft) est comprise entre 20 secondes et 10 minutes, de préférence entre 30 secondes et 5 minutes.
   - les moyens de mesure de teneur en CO₂ sont configurés pour opérer des mesures de concentration (i.e. teneur) en CO₂, au moins pendant la période de temps (dt) donnée.
   - l'IGU est configurée pour afficher au moins une valeur moyenne (Vmoy) de teneur en CO₂ fournie par les moyens de traitement de signal.
   - le CO₂ dont on mesure la valeur est produit par le patient, i.e. provenant des gaz expirés, et donc est observable lors de l'expiration du patient.
   - les moyens de mesure de teneur en CO₂ sont agencés de manière à opérer des mesures de concentration en CO₂ au niveau de la bouche du patient.
   - les moyens de mesure de teneur en CO₂ sont reliés électriquement aux moyens de traitement de signal, en particulier via une ou des liaisons électriques.
   - les moyens de mesure de teneur en CO₂ sont agencés sur le flux principal de gaz, c'est-à-dire qu'ils sont en agencement de type *'mainstream'* (i.e. flux principal).
   - alternativement, les moyens de mesure de teneur en CO₂ sont agencés dans l'appareil de monitorage, c'est-à-dire qu'ils sont en agencement de type 'sidestream', la mesure étant déportée dans l'appareil de monitorage.
   - les moyens de mesure de teneur en CO₂ comprennent un conduit de prélèvement de gaz dont une extrémité aval est agencée à proximité immédiate de la bouche du patient de manière à pouvoir déterminer la teneur en CO₂ dans les gaz expirés par le patient.
   - les moyens de mesure de teneur en CO₂ sont configurés pour opérer des mesures de préférence en continu.
   - les moyens de mesure de teneur en CO₂ comprennent un capteur de CO₂.
   - les moyens de mesure de teneur en CO₂ comprennent un capnomètre, c'est-à-dire un capteur de CO₂ ou tout autre dispositif de mesure de teneur en CO₂ analogue.
   - les moyens de mesure de teneur en CO₂ sont configurés pour opérer des mesures en continu.
   - les moyens de mesure de teneur en CO₂ sont configurés pour opérer des mesures de concentration en CO₂ successives sur des périodes de temps (dt) successives, c'est-à-dire des périodes de temps (dt) espacées les unes des autres.
   - les moyens de mémorisation coopérant avec les moyens de traitement de signal et de pilotage pour mémoriser les valeurs de teneur en CO₂ mesurées pendant chaque période de temps (dt) donnée et les valeurs maximales (Vmax) de teneur en CO₂ calculées pour chaque fenêtre de temps (Ft).
   - les moyens de traitement de signal comprennent au moins une carte électronique ou analogue.
   - les moyens de traitement de signal, en particulier la carte électronique, comprennent au moins un microprocesseur, de préférence un (ou plusieurs) microcontrôleur.
   - le microprocesseur met en oeuvre au moins un algorithme.
   - il comprend des moyens de mémorisation coopérant avec les moyens de traitement de signal pour mémoriser la pluralité de valeurs de teneur en CO₂ mesurées pendant la (ou les) période de temps donnée (dt).
   - les moyens de mémorisation sont en outre configurés pour mémoriser les valeurs maximales (Vmax) de teneur en CO₂ successives, c'est-à-dire mesurées pendant des périodes de temps (dt) données successives.
   - les moyens de mémorisation sont en outre configurés pour mémoriser des ou les valeurs moyennes (Vmoy) de teneur en CO₂ successives calculées à partir de valeurs maximales (Vmax) de teneur en CO₂ obtenues sur des fenêtres de temps (Ft) successives, de préférence une fenêtre de temps (Ft) glissante.
   - les moyens de traitement de signal sont configurés pour retrouver au sein des moyens de mémorisation, les valeurs de teneur en CO₂ ou les valeurs maximales (Vmax) et/ou moyennes (Vmoy) de teneur en CO₂ mémorisées.
   - les moyens de mémorisation comprennent une mémoire flash, par exemple de type disque dur ou autre.
   - les moyens de mémorisation sont agencés sur la carte électronique.
   - les moyens de mémorisation sont configurés de sorte que les valeurs de teneur en CO₂ enregistrées soient effacées :
      . soit après une durée non nulle préfixée par exemple comprise entre 2 et 10 secondes, de préférence entre 4 et 8 sec, avantageusement entre 5 et 7 sec,
      . soit lorsque les moyens de traitement détectent une valeur de CO₂ nulle parmi la pluralité de valeurs de CO₂ mémorisée, en particulier une ou plusieurs valeurs de CO₂ nulles pendant une durée entre 500 ms et 2 sec, de préférence moins de 1,5 secondes.
   - plusieurs valeurs maximales (Vmax) de teneur en CO₂ successives sont déterminées sur des périodes de temps (dt) successives, espacées les unes des autres d'une durée de pause (dp) comprise entre 200 ms à 3 secondes, typiquement entre 500 ms et 1,5 secondes.
   - l'IGU comprend un écran digital, de préférence un écran tactile.
   - l'affichage graphique sur l'IGU de la courbe de tendance représentant les variations de la teneur moyenne (Vmoy) en CO₂ est rafraîchi, c'est-à-dire mis à jour, après un intervalle de temps régulier et cyclique, typiquement après quelques secondes, par exemple après 2 à 20 secondes, de préférence moins de 10 secondes.
   - l'écran de l'IGU comprend plusieurs touches activant différentes fonctions et/ou plusieurs zones ou fenêtres d'affichage.
   - l'écran est du type à affichage en couleurs.
   - alternativement, l'écran est du type à affichage en noir et blanc.
   - alternativement, l'écran est du type permettant un choix ou passage d'un affichage en couleurs à un affichage en noir et blanc de manière à opérer des économies d'énergie, ou inversement.
   - il comprend une source de courant électrique, par exemple une batterie ou analogue, de préférence une batterie rechargeable.
   - il comprend des moyens d'alarme configurés pour se déclencher lorsque la valeur maximale (Vmax) ou moyenne (Vmoy) de teneur en CO₂ excède une valeur-seuil, notamment en cas de détection d'une (ou plusieurs) valeur maximale (Vmax) ou moyenne (Vmoy) de teneur en CO₂ correspondant à un RACS du patient.
   - les moyens d'alarme comprennent une alarme sonore ou visuelle, ou les deux.
   - les moyens d'alarme sont configurés pour alerter l'utilisateur en affichant un message d'alerte sur l'écran de l'IGU et/ou déclenchant une alerte sonore.
   - les moyens d'alarme sont configurés pour alerter l'utilisateur en affichant un message d'alerte après détection d'un (possible) RACS, par exemple en déclenchant une alerte spécifique de RACS ou en signalant le RACS sur un graphique affiché sur l'IGU.
   - les moyens d'alarme sont programmés pour se déclencher lorsque la valeur maximale (Vmax) de CO₂ mesurée, à un instant t, est telle que : [VmaxCO₂] > n x [MoyCO₂] , où:
      - n est compris entre 1,25 et 4, de préférence entre 1,5 et 3, par exemple de l'ordre de 2
      - [VmaxCO₂] est la valeur maximale de teneur en CO₂ mesurée pendant une durée dt donnée, par exemple sur une durée dt comprise entre 2 et 10 secondes, typiquement de l'ordre de 3 à 6 secondes,
      - [MoyCO₂] est la valeur moyenne des valeurs maximales de teneur en CO₂[VmaxCO₂] déterminées pour plusieurs durées dt successives comprises dans une fenêtre de temps (FT) donnée (FT> x.dt avec x ≥ 2), par exemple une période de 30 sec à 5 minutes, ou plus.
   - il comprend une carcasse ou coque externe rigide comprenant l'IGU, les moyens de traitement de signal, la source de courant électrique et les moyens de mémorisation.
   - la carcasse rigide est formée en tout ou en partie de polymère.
   - l'IGU est agencée dans l'une des parois formant la carcasse du ventilateur.
   - les moyens de mesure de l'activité cardiaque sont configurés pour opérer des mesures en continu.
   - les moyens de mesure de l'activité cardiaque du patient comprennent plusieurs électrodes venant se placer sur le corps du patient.
   - les moyens de mesure de l'activité cardiaque du patient comprennent plusieurs électrodes reliées électriquement aux moyens de traitement de signal et leur transmettant des signaux cardiaques, c'est-à-dire des signaux de mesure de l'activité cardiaque du patient, en particulier l'activité électrique du coeur.
   - les moyens de traitement de signal sont configurés pour traiter les signaux cardiaques provenant des moyens de mesure de l'activité cardiaque du patient.
   - l'interface graphique utilisateur est configurée pour afficher l'activité cardiaque du patient correspond aux signaux cardiaques traités, sous forme d'au moins une valeur numérique ou d'une représentation graphique, en particulier une courbe.
   - les moyens de mesure de l'activité cardiaque du patient sont configurés pour mesurer la fréquence cardiaque, le rythme cardiaque et/ou l'activité électrique cardiaque (ECG) du patient.
   - il comprend en outre des moyens de mesure de la saturation en oxygène (SpO₂) du patient, par exemple un capteur transcutané.
   - il comprend en outre des moyens de mesure de la température corporelle du patient, par exemple un capteur de température en contact avec le corps du patient.
   - il comprend en outre des moyens de mesure de la pression artérielle (PA), par exemple un brassard de mesure de pression artérielle.
   - les moyens de mesure de l'activité cardiaque du patient sont conçus pour pouvoir délivrer au moins un choc électrique au patient, via une ou des électrodes apposées sur le patient.
   - les moyens d'alarme sont programmés pour se déclencher une teneur en CO₂ très fiable ou nulle, correspondant à une mauvaise intubation du patient, une obstruction ou un débranchement du circuit patient.
   - il comprend en outre des moyens de mesure de l'activité électrique, c'est-à-dire de capter, grâce à des électrodes apposées sur le patient, le courant électrique généré par les cellules cardiaques et transmis à tout le corps et jusqu'à la peau, via les cellules corporelles qui sont conductrices d'électricité. Ainsi, un électrocardiogramme enregistre une succession de séquences de l'activité électrique du coeur, représentées par des ondes nommées P, QRS et T, où:
      - l'onde P est celle des oreillettes cardiaques, au moment de leur contraction,
      - l'ensemble QRS correspond à la contraction des ventricules du coeur,
      - l'onde T reflète la repolarisation (i.e. retour à la phase de repos) des ventricules.

Quand la formation ou la conduction de l'excitation électrique sont perturbées, on parle alors d'arythmies, également nommées troubles du rythme ou troubles de la conduction. L'activité électrique peut être normale (i.e. rythme sinusal) ou anormale (i.e. fibrillation, tachycardie, etc.) ou inexistante (i.e. tracé plat).
- il comprend en outre des moyens de défibrillation permettant de délivrer un choc électrique au patient, de préférence de manière manuelle ou semi-automatisée.
- les moyens de défibrillation comprennent au moins deux électrodes destinées à être placées sur le patient et reliées électriquement, par via un ou des câbles, circuit électrique ou analogues, à la carte électronique.
- les signaux de mesure provenant des électrodes sont analysés et/ou traitées par le microprocesseur des moyens de traitement de signal afin de piloter la délivrance d'un choc électrique au patient, ledit choc électrique étant ensuite délivré au patient via les électrodes.

En d'autres termes, la solution proposée par l'invention repose sur un appareil de monitorage ou moniteur cardiaque, par exemple un moniteur/défibrillateur, permettant de suivre un ou plusieurs paramètres ou signes vitaux du patient, notamment de l'activité électrique cérébrale du patient, et possédant par ailleurs des moyens de monitorage de la valeur moyenne (Vmoy) de teneur en CO₂ calculée à partir de plusieurs valeurs maximales (Vmax) de teneur en CO₂ successives, de préférence sur une fenêtre de temps glissante incluant une pluralité de durées dt successives, typiquement chaque durée dt est comprise entre 3 et 6 secondes environ.

Il permet ainsi de déterminer, à partir de la mesure instantanée de la teneur en CO₂, la concentration maximale ou Vmax généralement atteinte entre deux cycles inspiratoires, c'est-à-dire pendant la phase qui correspond à la phase d'expiration du patient et de visualiser facilement un RACS à partir de l'affichage graphique d'une courbe de tendance en CO₂, obtenue à partir des valeurs moyennes (Vmoy) de teneurs en CO₂.

En effet, la ventilation d'un patient en arrêt cardio-pulmonaire comprend une alternance entre une phase d'inspiration et une phase d'expiration. La ventilation est assurée grâce à des moyens d'insufflation de gaz de type ventilateur médical ou un dispositif de type BAVU à ballon auto-remplisseur et à valve unidirectionnelle couplé à une interface de type masque respiratoire, dispositif supra-glottique, sonde d'intubation ou autre ... L'appareil de monitorage cardiaque de l'invention est capable de déterminer la phase inspiratoire du patient à partir de la mesure de la teneur en CO₂, via un capnomètre par exemple, sachant que :
- pendant une/chaque phase inspiratoire, le gaz injecté au patient étant dépourvu de CO₂, la concentration détectée par le capteur de CO₂ de l'appareil de monitorage de l'invention est nulle,
- pendant une/chaque phase expiratoire, l'appareil de monitorage enregistre dans une mémoire, par exemple une mémoire flash, la valeur de concentration en CO₂ et détermine la valeur maximale Vmax atteinte.

La (ou les) valeur Vmax peut être affichée(s) sur l'écran de l'IGU sous forme numérique, c'est-à-dire de valeur, de graphique ou autre. Elles servent aussi à calculer des valeurs moyennes (Vmoy) de teneur en CO₂ au fil du temps.

En cas de retour à une activité cardiaque spontanée (RACS) du patient, la circulation sanguine du patient reprend brutalement et le CO₂ alvéolaire augmente parallèlement, ce qui induit une augmentation importante du CO₂ perçu par le moyen de mesure, c'est-à-dire d'un facteur souvent supérieur à 2. L'appareil de monitorage permet alors d'alerter l'utilisateur d'un possible RACS en déclenchant une alerte spécifique ou en signalant le possible RACS sur un graphique affiché sur l'IGU, grâce notamment à une représentation graphique, telle une courbe de tendance, des valeurs moyennes (Vmoy) de teneur en CO₂ au fil du temps.

De façon alternative, en cas de mauvaise intubation du patient, d'obstruction ou de débranchement du circuit patient, lorsque le patient est ventilé, la concentration en CO₂ sera nulle ou très faible. Dans ce cas, l'appareil de monitorage détecte alors cette teneur très fiable ou nulle et peut aussi alerter l'utilisateur via une alarme dédiée.

En particulier, l'appareil de monitorage ou moniteur cardiaque de l'invention est conçu pour suivre aussi l'activité électrique cardiaque du patient et des signes vitaux du type rythme et fréquence cardiaque...

L'appareil de monitorage cardiaque comprend aussi des moyens de mesure de l'activité électrique cardiaque du patient, notamment des électrodes placées sur le patient. Ces électrodes peuvent aussi faire office de moyens de défibrillation permettant de délivrer un choc électrique au patient, et ce, de manière manuelle ou semi-automatisée. Les électrodes sont placées sur le patient et reliées électriquement, par via un ou des câbles, circuit électrique ou analogues, au microprocesseur des moyens de traitement de signal où les signaux de mesure recueillis par lesdites électrodes sont analysés et traités afin notamment de piloter la délivrance d'un choc électrique au patient, via lesdites électrodes.

L'appareil peut aussi comprendre une interface homme-machine ou IHM, par exemple des boutons ou touches de sélection ou réglage, permettant de régler et/ou déclencher les chocs électriques.

Par ailleurs, l'appareil peut aussi comprendre des moyens de mesure de la pression artérielle du patient, de préférence de manière invasive via un cathéter ou analogue, de manière à pouvoir connaître la pression artérielle du patient.

L'invention ne concerne pas un procédé (i.e., une méthode) de monitorage, i.e. de suivi, d'une réanimation cardio-pulmonaire (RCP) opéré sur un patient en arrêt cardiaque, dans lequel :
- on met en oeuvre un appareil de monitorage tel que décrit ci-avant,
- on opère des mesures de concentration en CO₂ produit par ledit patient, par exemple au moyen d'un capnomètre,
- on traite les signaux de mesure de teneur en CO₂, par exemple au moyen de moyens de traitement de signal, tel un microprocesseur,
- on détermine une pluralité de valeurs de teneur en CO₂ mesurées pendant une période de temps (dt) donnée,
- on mémorise la pluralité de valeurs de teneur en CO₂ mesurées,
- on sélectionne la valeur maximale (Vmax) de teneur en CO₂ parmi la pluralité de valeurs de teneur en CO₂ mémorisées,
- on répète tout ou partie des étapes précédentes pour déterminer plusieurs valeurs maximales (Vmax) de teneur en CO₂ déterminées sur des périodes de temps (dt) successives, et
- on répète tout ou partie des étapes précédentes pour obtenir plusieurs valeurs maximales (Vmax) de teneur en CO₂ successives mesurées sur une fenêtre de temps (Ft) comprenant plusieurs périodes de temps (dt) successives, de préférence sur plusieurs fenêtres de temps (Ft) successives, typiquement sur une fenêtre de temps (Ft) glissante,
- on calcule au moins une valeur moyenne (Vmoy) de teneur en CO₂ à partir des valeurs maximales (Vmax) de teneur en CO₂ obtenues sur la fenêtre de temps (Ft), et
- on transmet ladite au moins une valeur moyenne (Vmoy) de teneur en CO₂ à l'interface graphique utilisateur (IGU).

Ensuite, on affiche sur l'IGU, plusieurs valeurs moyennes (Vmoy) de teneur en CO₂ obtenues successivement sous forme d'une représentation graphique, telle une courbe de tendance en fonction du temps.

L'invention va maintenant être mieux comprise grâce à la description détaillée suivante, faite à titre illustratif mais non limitatif, en référence aux figures annexées parmi lesquelles :
- la Figure 1 est un schéma d'un mode de réalisation d'un appareil de monitorage cardiaque selon l'invention, utilisable lors d'une RCP,
- la Figure 2 qui schématise l'évolution de l'etCO₂ dans les gaz expirés par le patient sur plusieurs périodes de temps successives,
- la Figure 3 schématise la quantité de CO₂ dans les gaz expirés, avant et lors du retour à une activité cardiaque spontanée (RACS) du patient,
- la Figure 4 schématise la quantité de CO₂ mesurée par l'appareil de l'invention de la Figure 1, pendant une RCP, et au moment et après retour à une activité cardiaque spontanée (RACS), et
- la Figure 5 schématise les mesures et les intervalles de temps utilisés pour le calcul et l'affichage de la tendance en CO₂, c'est-à-dire des valeurs moyennes (Vmoy) de CO₂ au fil du temps.

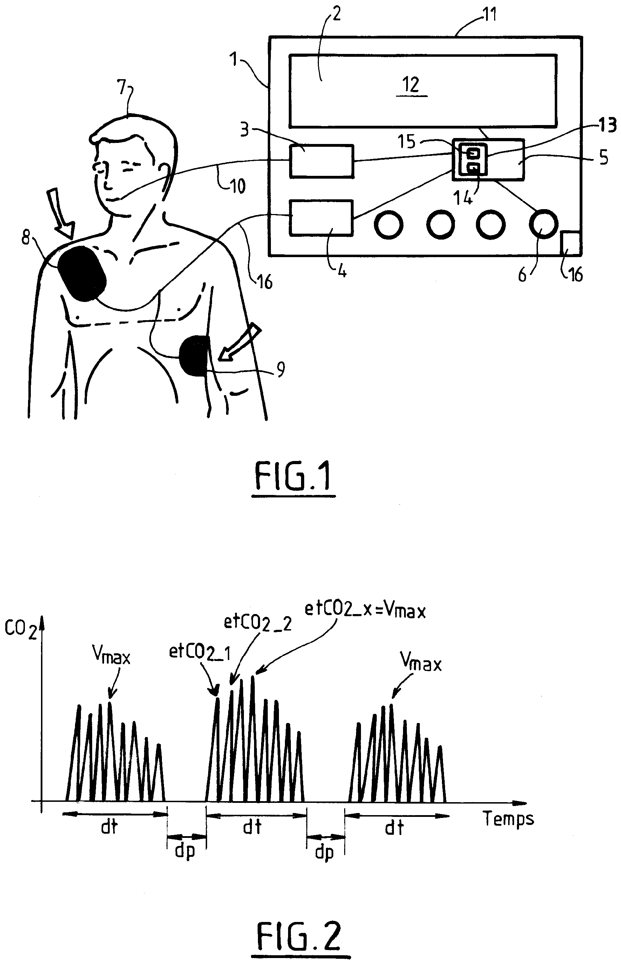

La Figure 1 est une représentation schématique d'un mode de réalisation d'un appareil médical de monitorage cardiaque 1 selon l'invention permettant de monitorer un ou des signes ou paramètres vitaux, en particulier l'activité électrique du coeur, ainsi que la teneur en CO₂ dans les gaz expirés, chez un patient 7 en ACR, en particulier avant, pendant et/ou après une RCP. L'appareil de monitorage cardiaque de l'invention ne comprend pas de moyens d'insufflation de gaz, telle une micro-soufflante ou analogue.

Le moniteur 1 est commandé, c'est-à-dire piloté, par des moyens de traitement de signal 5 comprenant une carte électronique 13 à (un ou plusieurs) microprocesseur 14, en particulier à microcontrôleur, mettant en oeuvre un ou plusieurs algorithmes. Il comprend en outre des moyens de mémorisation 15, telle une carte mémoire ou analogue, de préférence ils sont portés par la carte électronique 13.

Par ailleurs, l'appareil de monitorage 1 comprenant des moyens de mesure de l'activité cardiaque 4 du patient comprenant deux électrodes 8, 9 apposées sur le patient 7 et reliées électriquement aux moyens de traitement de signal 5, via un ou des câbles électriques 16. Les moyens de mesure de l'activité cardiaque 4 du patient permettent de mesurer notamment l'activité électrique cardiaque du patient et la fréquence cardiaque à partir des données recueillies par les deux électrodes 8, 9.

Les mesures (signaux) sont transmises électriquement aux moyens de traitement de signal 5 et à une interface graphique utilisateur ou IGU 2 qui est conçue pour afficher ces mesures sous forme de valeurs numériques et/ou de représentations graphiques, telles des courbes d'évolution au fil du temps.

Par ailleurs, on prévoit aussi des moyens de mesure 3 de teneur en CO₂, tel un capnomètre, pour opérer des mesures de concentration, i.e. teneur, en CO₂ présents dans les gaz expirés par le patient et transmettre les signaux de mesure aux moyens de traitement de signal 5.

Les moyens de traitement de signal 5 conçus pour traiter, au sein de leur microprocesseur 14, les signaux de mesure de teneur en CO₂ provenant des moyens de mesure de teneur en CO₂ 3 et obtenir une pluralité de valeurs de teneur en CO₂ mesurées pendant la période de temps (dt) donnée, typiquement entre 3 et 6 secondes. Les valeurs de teneur en CO₂ sont mémorisées, i.e. enregistrées, par les moyens de mémorisation 15.

Ensuite, les moyens de traitement de signal 5 sélectionnent la valeur maximale (Vmax) de teneur en CO₂ parmi la pluralité de valeurs de teneur en CO₂ mesurées pendant la période de temps (dt) donnée et mémorisées par les moyens de mémorisation 15, et transmettent cette valeur maximale (Vmax) de teneur en CO₂ à l'IGU 2 pour que cette dernière puisse alors afficher cette valeur maximale (Vmax) de teneur en CO₂, par exemple sur un écran d'affichage digital et tactile.

Préférentiellement, plusieurs valeurs maximales (Vmax) de teneur en CO₂ sont déterminées sur plusieurs périodes de temps (dt) données successives au fil du temps, typiquement quelques secondes, par exemple des périodes de temps (dt) entre 2 et 6 secondes. Cette succession de valeurs maximales (Vmax) de teneur en CO₂ peuvent être mémorisées et avantageusement affichées sur l'IGU 2, notamment d'une courbe, un barre-graphe ou tout autre représentation graphique au fil du temps.

Les moyens de mémorisation, telle une mémoire de type disque dur, mémoire flash ou autres permettent de mémoriser les valeurs mesurées, notamment celles de teneur en CO₂ et/ou les valeurs Vmax obtenues. Des seuils peuvent aussi être mémorisés et des alarmes déclenchées en cas de franchissement de ces seuils.

L'écran de l'IGU 2 permet donc d'afficher graphiquement les informations traitées par le processeur 14 des moyens de traitement de signal 5, sous forme par exemple d'une courbe d'activité électrique cardiaque, une fréquence cardiaque, une fréquence respiratoire, une courbe de capnographie (i.e., teneur en CO₂), la ou les teneurs en CO₂ maximale (Vmax)...

Avantageusement, les électrodes 8, 9 ou d'autres électrodes dédiées peuvent également être utilisées pour délivrer un choc électrique au patient, appelé « défibrillation ». En particulier, le moniteur 1 peut exploiter la mesure du CO₂, au sein de son microprocesseur 14, pour délivrer un choc électrique à un moment choisi du cycle de ventilation, par exemple pendant la phase expiratoire ou lors d'un pic de CO₂.

Les différents réglages peuvent être opérés par l'utilisateur au moyen d'une interface homme-machine ou IHM 6 comprend des moyens de réglage, tel que boutons, touches, curseurs etc...

Plus précisément, les moyens de mesure de teneur en CO₂ 3, aussi appelés capteur de CO₂ ou capnomètre, sont conçus pour opérer des mesures de concentration en CO₂ dans le gaz expiré par le patient P, et fournir des signaux de mesure de teneur en CO₂ au microprocesseur 14 des moyens de traitement de signal 5 où ces signaux de mesure peuvent être traités, notamment par un (ou des) algorithme de calcul ou analogue. Préférentiellement, le capteur de CO₂ opère des mesures en continu de la concentration en CO₂. Les signaux de mesure de teneur en CO₂ sont ensuite transmis par le capteur de CO₂, par liaison électrique ou analogues, notamment filaire ou autre, aux moyens de traitement de signal 5.

Selon l'invention, le monitorage de la teneur en CO₂, en particulier de l'etCO₂ qui reflète indirectement le CO₂ alvéolaire, est d'une grande importance pendant la RCP, notamment pour détecter un retour d'activité cardiaque spontanée ou RACS. En effet, un RACS engendre une augmentation significative du débit cardiaque, suivie d'une augmentation rapide de la quantité de CO₂ amenée par le sang aux poumons et transférée à travers la membrane alvéolo-capillaire.

Le CO₂ se retrouve ensuite dans le flux gazeux expiré par le patient et toute augmentation significative de quantité de CO₂ dans le gaz expiré peut alors être détectée grâce à la détermination et au suivi des valeurs maximales (Vmax) de CO₂ sur des périodes de temps dt données, typiquement des périodes successives de moins de 6 secondes.

Pour ce faire, les moyens de traitement de signal 5 sont configurés, en particulier leur microprocesseur 14, pour :
- traiter les signaux de mesure de teneur en CO₂ qui correspondent à des mesures opérées par les moyens de mesure de teneur en CO₂ 3, typiquement le capnomètre ou capteur de CO₂, en particulier pendant la période de temps considérée (dt), par exemple quelques secondes, pour en extraire une pluralité de valeurs de teneur en CO₂, lesquelles sont avantageusement mémorisées par les moyens de mémorisation 15 portés par exemple par la carte électronique 13,
- sélectionner la valeur maximale (Vmax) de teneur en CO₂ parmi ladite pluralité de valeurs de teneur en CO₂ mesurées et mémorisées pendant la période de temps considérée (dt), et
- répéter plusieurs fois les étapes précédentes, pendant plusieurs périodes de temps (dt) successives, de manière à obtenir plusieurs valeurs maximales (Vmax) de teneur en CO₂ successives,
- utiliser ces valeurs maximales (Vmax) de teneur en CO₂ successives pour calculer des valeurs moyennes (Vmoy) de teneur en CO₂, et
- transmettre les valeurs moyennes (Vmoy) de teneur en CO₂ obtenues à l'IGU 2 où elles sont affichées graphiquement sous forme d'une courbe de tendance par exemple.

Une source de courant électrique 16, telle une batterie rechargeable ou analogue, alimente, directement ou indirectement, en courant électrique le moniteur 1, en particulier les moyens de traitement de signal 5, l'IGU 2, les moyens de mesure de l'activité cardiaque 4 du patient, en particulier les deux électrodes 8, 9, et tout autre élément du moniteur 1 qui nécessitent de l'énergie électrique pour fonctionner, notamment la carte électronique 13, le processeur 14, les moyens de mémorisation 15, l'IHM 2... La source de courant électrique 16 est préférentiellement agencée, de manière extractible ou non, dans la carcasse 11 rigide du moniteur 1.

D'une façon générale, le moniteur médical 1 de l'invention permet une mesure en continu de la concentration en CO₂ produit par le patient P, ainsi qu'un ou plusieurs signes vitaux cardiaques du patient, telle son activité électrique cardiaque.

Selon l'invention, l'IGU 2 est donc configurée pour afficher non seulement ce ou ces signes vitaux cardiaques mais aussi la courbe de tendance de valeurs moyennes (Vmoy) de teneur en CO₂ fournies par les moyens de traitement de signal 5.

D'une façon générale, la valeur de concentration en CO₂ qui reflète le plus la teneur en CO₂ alvéolaire, et donne dès lors une bonne indication de l'état de la circulation sanguine chez le patient P durant la RCP, est la valeur la plus haute de CO₂, encore appelée valeur maximale (Vmax) ou valeur de pic, comme illustré en Figure 2.

Plus précisément, la Figure 2 schématise l'évolution de la teneur en CO₂ dans le gaz. On y a représenté plusieurs mesures de l'etCO₂ mesurées pendant plusieurs durées dt successives, par exemple des durées de 3 à 6 secondes, pendant la réalisation d'une RCP.

On y voit que la teneur en CO₂ du gaz n'est pas constante pendant un intervalle de temps dt donné et qu'il existe donc forcément une valeur maximale (Vmax) de teneur en CO₂ sur chaque intervalle dt.

Dans le cadre de la présente invention, le moniteur 1 mémorise toutes les valeurs de pics de CO₂ pendant chaque période de temps dt successives considérées et détermine la valeur maximale Vmax de teneur en CO₂ parmi la pluralité de pics (EtCO2_{_1}, EtCO2_{_2}, EtCO2_{_3}, ..., EtCO2_x) mesurés sur cette période de temps (dt) considérée.

Le processeur 14 peut alors retrouver parmi la pluralité de pics (EtCO2_{_1}, EtCO2_{_2}, EtCO2_{_3}, ..., EtCO2_x) mémorisés, la valeur maximale Vmax de teneur en CO₂ durant la période de temps dt considérée.

On peut répéter ces opérations plusieurs fois de suite sur des périodes de temps dt successives comme illustré en Figure 2. De préférence, plusieurs valeurs maximales (Vmax) de teneur en CO₂ successives sont déterminées sur des périodes de temps dt successives, espacées les unes des autres d'une durée de pause (dp) comprise entre 200 ms à 3 secondes, typiquement entre 500 ms et 1,5 secondes.

La ou de préférence les valeurs maximales Vmax de teneur en CO₂ peuvent alors être éventuellement affichées sur l'IGU 2, par exemple en fonction du temps, par exemple sous forme d'une courbe ou d'un barre-graphe. Toutefois, dans le cadre de l'invention, on les utilise pour calculer des valeurs moyennes Vmoy de teneur en CO₂, lesquelles sont ensuite transmises et affichées sur l'IGU 2, notamment sur un écran 12 digital, sous la forme d'une courbe de tendance qui est régulièrement « rafraîchie », par exemple après 2 à 10 ou 15 sec environ.

On peut prévoir par ailleurs que les moyens de mémorisation 15 soient configurés pour que les valeurs de teneur en CO₂ enregistrées soient effacées :
- soit après une durée non nulle préfixée par exemple comprise entre 2 et 10 secondes, de préférence entre 4 et 8 sec, avantageusement entre 5 et 7 sec,
- soit lorsque les moyens de traitement 5 détectent une valeur de CO₂ nulle parmi la pluralité de valeurs de CO₂ mémorisée, en particulier une ou plusieurs valeurs de CO₂ nulles pendant une durée entre 500 ms et 2 sec, de préférence moins de 1,5 secondes.

La valeur maximale de CO₂ (Vmax) pour chaque période dt est celle qui représente le mieux le CO₂ alvéolaire lors d'une RCP. En effet, le CO₂ présent au niveau du capnomètre 3 est "lavé" petit à petit du fait des CT successives et répétées, et tend à diminuer après avoir atteint cette valeur maximale puisque les CT engendrent aussi l'évacuation vers l'atmosphère (des gaz riches en CO₂, via la branche expiratoire de l'appareil de ventilation ou du BAVU utilisé pour ventiler le patient pendant la RCP. Les CT successives génèrent donc différents niveaux de CO₂, la plus représentative étant la valeur de pic ou maximale (Vmax).

Dit autrement, dans le cadre de la présente invention, le moniteur 1 mémorise toutes les valeurs maximales (Vmax) de CO₂ entre deux cycles ventilatoires, c'est-à-dire pendant les durées dt successives, détermine la valeur maximale (Vmax) de teneur en CO₂ parmi la pluralité de valeurs maximales mesurés, c'est-à-dire les pics de CO₂.

Les données calculées, c'est-à-dire les valeurs Vmoy, et la courbe de tendance obtenue à partir de ces mesures de CO₂ permettent au secouriste de mieux « piloter » la RCP grâce à un indicateur qui reflète l'état de la circulation et du métabolisme du patient puisque, à un niveau de ventilation constant, plus la RCP est efficace plus la quantité de CO₂ produite et transférée à travers la membrane alvéolo-capillaire est importante et donc plus la quantité de CO₂ pouvant être détectée au niveau du capnomètre 3 est importante.

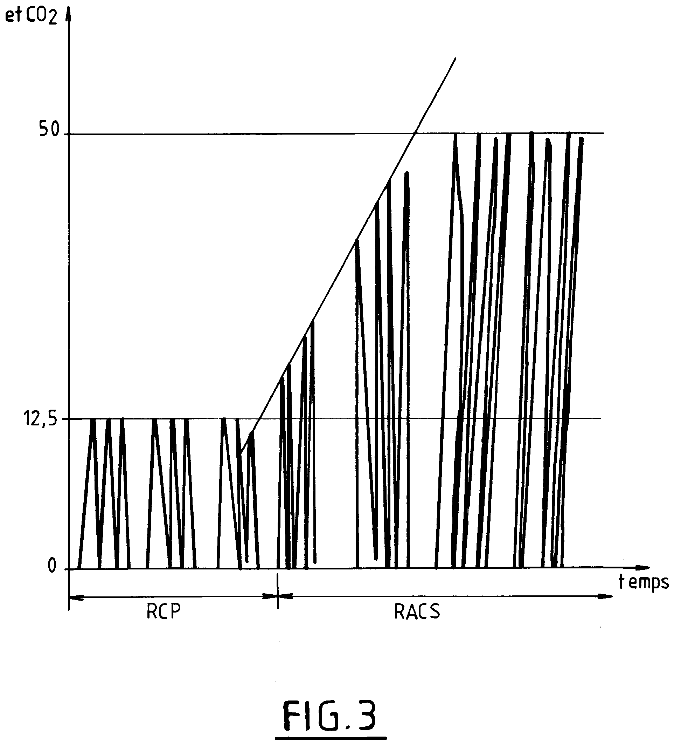

De là, en cas de retour à une activité cardiaque spontanée (RACS), la circulation reprend brutalement et donc la quantité de CO₂ alvéolaire augmente parallèlement, ce qui induit une augmentation importante la quantité de CO₂ perçu par le capnomètre d'un facteur souvent supérieur à 2, comme illustré sur la Figure 3.

Ainsi, on voit sur la Figure 3 que l'EtCO₂ est toujours inférieure à 25 KPa pendant la RCP, alors que l'EtCO₂ augmente subitement jusqu'à atteindre plus de 50 KPa lors du RACS. Ceci peut être immédiatement détecté visuellement sur l'écran 12 de l'IGU 2 par le secouriste qui peut alors réaliser une analyse de rythme pour stopper le massage cardiaque en cas de RACS effectif.

Dit autrement, dans le cadre de l'invention, le fait d'afficher sur l'IGU 2, une courbe de tendance de valeurs moyennes de CO₂ permet au secouriste de mieux détecter la survenance du RACS puisque cette courbe reflète étroitement le CO₂ alvéolaire.

En effet, il a été constaté, dans le cadre d'essais qu'afficher en continu toutes les mesures de CO₂, comme proposé dans l'état de la technique, ne serait pas efficace car un massage cardiaque, même pratiqué de manière régulière (force de pression, fréquence...), engendre inévitablement des variations importantes de teneur en CO₂ au niveau du capnomètre, d'une CT à une autre. Cela s'expliquant par le comportement dynamique, d'ouverture/fermeture des petites voies aériennes ainsi que par l'effet de lavage de l'espace mort lors des compressions thoraciques successives entre deux cycles machine.

Dès lors, afficher toutes les mesures de CO₂ pourrait induire le secouriste en erreur ou encore le « noyer » sous trop d'informations et celui-ci pourrait alors parfois « croire » à un RACS alors qu'il ne s'agirait que d'un artefact, ou à l'inverse, ne pas s'apercevoir d'un RACS chez le patient et continuer le massage alors que le patient serait en phase de RACS. Dans tous les cas, l'utilisation d'une unique valeur instantanée à des fins de pronostic ou de choix de stratégie thérapeutique est rendu risqué par le caractère oscillant de la valeur d'etCO2 instantanée, i.e. à chaque compression thoracique (CT).

Dans le cadre de l'invention, il a été montré que ces problèmes pouvaient être totalement surmontés en n'affichant que la valeur la plus élevée de teneur en CO₂ (Vmax) correspondant une période de temps (dt) donnée, typiquement de quelques secondes, et préférence plusieurs Vmax successives, mais plus préférentiellement en affichant une courbe de tendance sur une fenêtre de temps glissante Ft, de plusieurs valeurs moyennes (Vmoy) de teneur en CO₂ qui sont obtenues à partir d'une succession de plusieurs teneurs en CO₂ (Vmax) obtenues sur des durées dt successives durant la fenêtre de temps glissante Ft considérée.

En outre, il a été constaté que la teneur en CO₂ mesurée à chaque compression thoracique peut varier énormément d'une compression thoracique à l'autre. Ceci est dû non seulement à l'espace mort anatomique et instrumental mais aussi au degré d'ouverture des voies aériennes du patient. En prenant en compte ces facteurs, la valeur maximale (Vmax) de CO₂ apparaît être dès lors un meilleur reflet du CO₂ alvéolaire, et est donc un bon indicateur de RACS (si elle augmente brutalement) ou de nouvel arrêt cardiaque (si elle diminue brutalement), ce qui informe immédiatement le secouriste et de manière plus pertinente.

Ainsi, lorsque le secouriste constate une forte élévation de la valeur en CO₂ affichée, en particulier la courbe de tendance basée sur les valeurs moyennes (Vmoy) de teneurs en CO₂ déterminées, celui-ci peut en conclure que le patient est en phase de RACS et peut alors décider de stopper le massage cardiaque afin de procéder à une analyse de rythme par exemple.

Avantageusement, le moniteur 1 de l'invention peut aussi inclure des moyens d'alarme conçue et programmée pour avertir le secouriste ou analogue lorsque la valeur maximale de CO₂ mesurée excède ou, à l'inverse, devient inférieure une valeur-seuil donnée, préfixée ou calculée en continu.

En particulier, on prévoit une alarme sonore et/ou visuelle qui se déclenche lorsque la valeur maximale de CO₂ mesurée, à un instant t, est supérieure à une valeur-seuil, par exemple :
[VmaxCO₂] > 1,5 x [MoyCO₂] où :
- [VmaxCO₂] est la valeur maximale de teneur en CO₂ mesurée pendant une durée dt donnée, par exemple sur une durée dt comprise entre 2 et 10 secondes,
- [MoyCO₂] est la valeur moyenne des valeurs maximales de teneur en CO₂ [VmaxCO₂] déterminées pour plusieurs durées dt successives comprises dans une fenêtre de temps (FT) donnée (FT> x.dt avec x ≥ 2), par exemple une période de 30 sec à 5 minutes, ou plus.

De façon analogue, l'alarme peut se déclencher en cas de chute brutale de la concentration en CO₂ sous une valeur minimale donnée qui pourrait être le signe d'un nouvel arrêt cardiaque du patient, d'une hyperventilation ou d'une obstruction du circuit de gaz entre le patient et la machine, par exemple un conduit flexible plié ou écrasé et ne laissant plus passer le gaz.

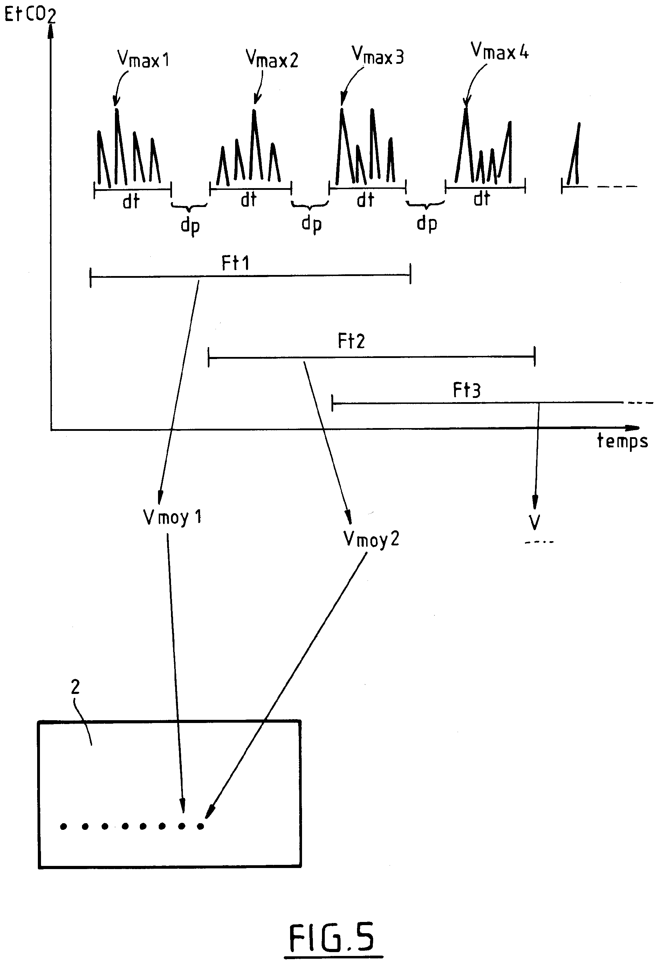

Autrement dit, selon la présente invention et comme illustré en Figure 5, les moyens de traitement de signal 5 sont en outre configurés, en particulier le microprocesseur, pour :
- déterminer plusieurs valeurs maximales (Vmax1, Vmax2, Vmax3...) de teneur en CO₂ successives sur des périodes de temps dt successives, par exemple comprises entre 2 et 10 sec, typiquement de l'ordre de 3 à 6 sec, espacées les unes des autres d'une durée de pause (dp) comprise entre 200 ms à 3 secondes, typiquement entre 500 ms et 1,5 secondes, en répétant autant de fois que nécessaire ce qui est décrit ci-dessus, et ce, sur une fenêtre de temps (Ft1, Ft2, Ft3....) comprenant plusieurs périodes de temps (dt) successives, par exemple une fenêtre de temps de 20 secondes à 10 minutes, de préférence entre 30 secondes et 5 minutes.
- calculer des valeurs moyennes (Vmoy1, Vmoy2,...) de teneur en CO₂ à partir des valeurs maximales (Vmax1, Vmax2, Vmax3...) de teneur en CO₂ obtenues sur chaque fenêtre de temps (Ft1, Ft2, Ft3..), et
- transmettre les valeurs moyenne (Vmoy1, Vmoy2,...) de teneur en CO₂ à l'IGU 2.

De préférence, on calcule plusieurs valeurs moyennes (Vmoy1, Vmoy2,...) de teneur en CO₂ successives à partir des valeurs maximales (Vmax1, Vmax2, Vmax3...) de teneur en CO₂ obtenues sur des fenêtres de temps (Ft1, Ft2, Ft3..) successives, de préférence une fenêtre de temps (Ft) glissante au fil du temps.

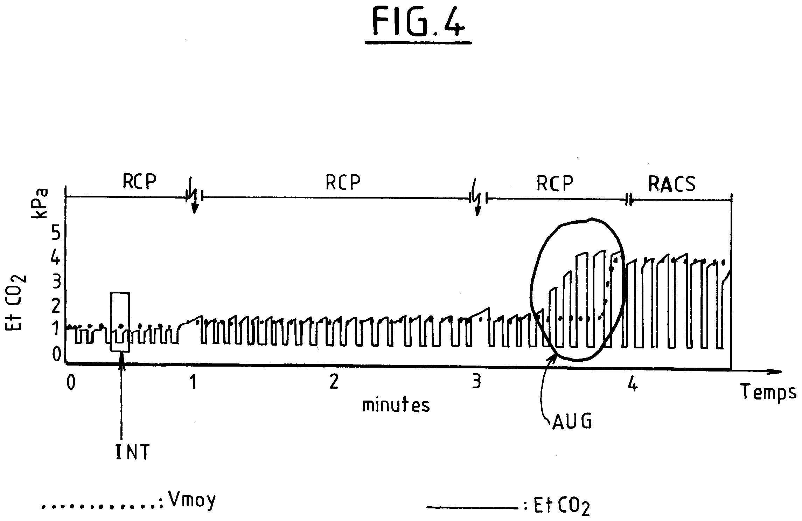

L'IGU 2 va alors pouvoir afficher au ces valeurs moyennes (Vmoy1, Vmoy2,...) successives de teneurs en CO₂ sous forme de valeurs numériques et/ou d'une représentation graphique, de préférence sous la forme d'une courbe formée d'une succession de symboles graphiques (e.g. points, triangles, carrés, croix.... ou d'autres symboles reliés ou non entre eux), chaque symbole graphique correspondant à une valeur moyenne (Vmoy ; Vmoy1, Vmoy2,...) de teneur en CO₂, comme illustré en Figures 4 et 5.

Bien entendu, d'autres types d'affichage sont possibles, comme par exemple un barre-graphe comprenant plusieurs barres, chaque barre correspondant à une valeur moyenne (Vmoy ; Vmoy1, Vmoy2,...) de teneur en CO₂.

L'IGU 2 permet donc d'afficher un graphique temporel représentant les variations de valeur moyenne (Vmoy) de teneur en CO₂ en fonction du temps, c'est-à-dire une courbe de tendance ou analogue.

Le calcul des valeurs moyennes (Vmoy) de teneur en CO₂ se fait à partir des valeurs maximales (Vmax) de teneur en CO₂ obtenues sur les fenêtres de temps (Ft1, Ft2, Ft3..) successives, typiquement une fenêtre de temps glissante (Ft), qui ont été mémorisées pour la fenêtre de temps (Ft) considérée, au sein des moyens de mémorisation 15. Les valeurs maximales (Vmax) de teneur en CO₂ utilisées sont retrouvées au sein des moyens de mémorisation 15 par les moyens de traitement de signal 5.

La (ou les) valeur moyenne (Vmoy) de CO₂ affichée n'est pas obligatoirement mise à jour lors de l'affichage de chaque point, mais peut être rafraîchie et affichée après une durée définie, par exemple quelques secondes. Comme déjà expliqué, la valeur de concentration en CO₂ qui reflète le plus la teneur en CO₂ alvéolaire, et qui donne dès lors une bonne indication de l'état de la circulation sanguine chez le patient P durant la RCP, est la valeur la plus haute de CO₂, encore appelée valeur de pic maximale, comme illustré en Figure 2.

Un affichage des valeurs moyennes (Vmoy) de teneur en CO₂ au fil du temps permet de suivre l'évolution de la teneur en CO₂ dans le flux gazeux sortant des poumons du patient pendant le massage cardiaque, en particulier sous l'effet des CTs.

Ainsi, sur la Figure 4, la courbe « ...... » représente les valeurs de Vmoy et la courbe « _ » représente les valeurs de Et-CO2.

Comme on le voit, la courbe des valeurs moyennes Vmoy de CO₂ constitue un indicateur très utile au secouriste puisqu'elle lui permet visualiser ce qui se passe au niveau physiologique chez le patient et donc de mieux conduire la RCP. En effet, cette courbe des valeurs moyennes Vmoy de CO₂ reflète l'état de la circulation et du métabolisme du patient à partir du moment où le patient est intubé (INT) et qu'une RCP est pratiquée. Plus la RCP est efficace plus la quantité de CO₂ produite et transférée à travers la membrane alvéolo-capillaire est importante, et donc plus la quantité de CO₂ pouvant être détectée au niveau du capnomètre est importante.

De là, comme visible sur la Figure 4, en cas de retour à une activité cardiaque spontanée (RACS), la circulation reprend brutalement (zone encerclée) et donc la quantité de CO₂ alvéolaire augmente parallèlement, ce qui induit une augmentation importante la quantité de CO₂ perçu par le capnomètre d'un facteur souvent supérieur à 2, comme illustré sur la Figure 4. En effet, on voit sur la Figure 4 que l'EtCO₂ est toujours inférieure à 2.5 pendant la RCP, mais qu'elle augmente (AUG) subitement jusqu'à atteindre plus de 5 au moment du RACS, soit après environ 3 à 4 minutes après l'intubation (INT) du patient et le début de la RCP.

Dans le cadre de l'invention, le fait d'afficher sur l'IGU 2, une courbe de tendance basée sur les valeurs moyennes Vmoy déterminées sur une fenêtre de temps (Ft) glissante permet au secouriste de mieux détecter la survenance du RACS puisque la courbe Vmoy fait apparaître une forte augmentation (AUG sur Fig. 4) au moment d'un RACS du fait de la libération accrue de CO₂ par les poumons se retrouvant dans les gaz expirés.

Dès lors, lorsque le secouriste constate une forte élévation (AUG) de la courbe de valeurs moyennes Vmoy en CO₂ sur l'IGU 2, celui-ci peut en conclure que le patient est début de RACS et peut par exemple décider d'analyser le rythme cardiaque et éventuellement de stopper le massage cardiaque.

L'appareil de monitorage selon la présente invention est particulièrement adapté à une mise en oeuvre pendant une réanimation cardio-pulmonaire (RCP) sur une personne (i.e. un patient) en arrêt cardio-pulmonaire, dans le cadre de laquelle un gaz respiratoire, tel de l'air sous pression, est fourni selon un cycle ventilatoire à plusieurs niveaux de pression à ladite personne subissant le massage cardiaque, par le biais d'un ventilateur médical ou d'un BAVU.

Pour faciliter son transport par les secouristes, par exemple un médecin, un infirmier, un pompier ou analogue, l'appareil de monitorage de l'invention est préférentiellement agencé dans un sac de transport et/ou muni d'une poignée de transport ou analogue.

## Revendications

1. Appareil de monitorage (1) cardiaque d'un patient, en particulier d'un patient en arrêt cardio-respiratoire (ACR) pendant une réanimation cardio-pulmonaire (RCP), comprenant :
- des moyens de mesure de l'activité cardiaque (4, 8, 9) du patient,
- au moins une interface graphique utilisateur (2),
- des moyens de mesure de teneur en CO₂ (3) pour opérer des mesures de concentration en CO₂ produit par ledit patient et fournir des signaux de mesure de teneur en CO₂ à des moyens de traitement de signal (5),
- des moyens de traitement de signal (5) configurés pour traiter les signaux de mesure de teneur en CO₂ provenant des moyens de mesure de teneur en CO₂ (3), et
- des moyens de mémorisation (15) pour enregistrer au moins les valeurs de CO₂ traitées,
**caractérisé en ce que** :
A) les moyens de traitement de signal (5) sont configurés pour :
i) sélectionner une valeur maximale (Vmax) de teneur en CO₂ parmi une pluralité de valeurs de teneur en CO₂ mesurées pendant une période de temps (dt) donnée, et enregistrées dans les moyens de mémorisation (15),
ii) répéter l'étape i) pour obtenir plusieurs valeurs maximales (Vmax) de teneur en CO₂ successives mesurées sur une fenêtre de temps (Ft) comprenant plusieurs périodes de temps (dt) successives,
iii) calculer au moins une valeur moyenne (Vmoy) de teneur en CO₂ à partir des valeurs maximales (Vmax) de teneur en CO₂ obtenues sur la fenêtre de temps (Ft),
iv) répéter les étapes i) à iii) de manière à obtenir plusieurs valeurs moyennes (Vmoy) de teneur en CO₂ successives calculées à partir de valeurs maximales (Vmax) de teneur en CO₂ obtenues sur plusieurs fenêtres de temps (Ft) successives, et
v) transmettre les valeurs moyennes (Vmoy) de teneur en CO₂ à l'interface graphique utilisateur (2), et
B) l'interface graphique utilisateur (2) est configurée pour afficher les valeurs moyennes (Vmoy) de teneur en CO₂ sous forme d'au moins une représentation graphique.

2. Appareil selon la revendication 1, **caractérisé en ce que** les fenêtres de temps (Ft) successives sont une fenêtre de temps (Ft) glissante.

3. Appareil selon l'une des revendications 1 ou 2, **caractérisé en ce que** l'interface graphique utilisateur (2) est configurée pour afficher au moins une partie des valeurs moyennes (Vmoy) successives de teneur en CO₂ calculées sous forme :
- d'une courbe formée d'une succession de symboles graphiques, chaque symbole graphique correspondant à une valeur moyenne (Vmoy) de teneur en CO₂, ou
- d'un barre-graphe comprenant plusieurs barres, chaque barre dudit barre-graphe correspondant à une valeur moyenne (Vmoy) de teneur en CO₂.

4. Appareil selon l'une des revendications 1 ou 2, **caractérisé en ce que** :
- la période de temps (dt) est comprise entre 2 et 10 secondes, typiquement de l'ordre de 3 à 6 secondes, et/ou
- la fenêtre de temps (Ft) est comprise entre 20 secondes et 10 minutes, de préférence entre 30 secondes et 5 minutes.

5. Appareil selon l'une des revendications précédentes, **caractérisé en ce que** les moyens de mémorisation (15) sont configurés de sorte que les valeurs de teneur en CO₂ enregistrées soient effacées :
- soit après une durée non nulle préfixée par exemple comprise entre 2 et 10 secondes,
- soit lorsque les moyens de traitement détectent une valeur de CO₂ nulle parmi la pluralité de valeurs de CO₂ mémorisée, en particulier une ou plusieurs valeurs de CO₂ nulles pendant une durée entre 500 ms et 2 sec.

6. Appareil selon l'une des revendications précédentes, **caractérisé en ce que** plusieurs valeurs maximales (Vmax) de teneur en CO₂ successives sont déterminées sur des périodes de temps (dt) successives, espacées les unes des autres d'une durée de pause (dp) comprise entre 200 ms à 3 secondes.

7. Appareil selon l'une des revendications précédentes, **caractérisé en ce que** l'IGU comprend un écran (12) digital.

8. Appareil selon l'une des revendications précédentes, **caractérisé en ce que** l'affichage graphique sur l'IGU (2) de la courbe de tendance représentant les variations de la teneur moyenne (Vmoy) en CO₂ est rafraîchi après un intervalle de temps régulier et cyclique, de préférence après quelques secondes.

9. Appareil selon l'une des revendications précédentes, **caractérisé en ce que** l'IGU (2) est configurée pour afficher une courbe de tendance formée d'une succession de symboles graphiques correspondant chacun à une valeur moyenne (Vmoy) de teneur en CO₂ sur une fenêtre de temps glissante (Ft) comprise entre 30 sec et 5 minutes, typiquement de 1 à 3 minutes.

10. Appareil selon l'une des revendications précédentes, **caractérisé en ce qu'**il comprend des moyens d'alarme configurés pour se déclencher lorsque la valeur maximale (Vmax) ou moyenne (Vmoy) de teneur en CO₂ excède une valeur-seuil correspondant à un RACS du patient.

11. Appareil selon l'une des revendications précédentes, **caractérisé en ce que** :
- les moyens de traitement de signal (5) sont configurés en outre pour traiter les signaux cardiaques provenant des moyens de mesure de l'activité cardiaque (4, 8, 9) du patient, et
- l'interface graphique utilisateur (2) est configurée pour afficher l'activité cardiaque du patient correspond aux signaux cardiaques traités, sous forme d'au moins une valeur numérique ou d'une représentation graphique, en particulier une courbe.

12. Appareil selon l'une des revendications précédentes, **caractérisé en ce que** les moyens de mesure de l'activité cardiaque (4, 8, 9) du patient sont configurés pour mesurer la fréquence cardiaque, le rythme cardiaque et/ou l'activité électrique cardiaque (ECG) du patient, de préférence les moyens de mesure de l'activité cardiaque (4, 8, 9) du patient comprennent plusieurs électrodes (8, 9) venant se placer sur le corps du patient.

13. Appareil selon l'une des revendications précédentes, **caractérisé en ce que** les moyens de mesure de teneur en CO₂ (3) comprennent un capnomètre.

14. Appareil selon l'une des revendications précédentes, **caractérisé en ce qu'**il comprend en outre :
- des moyens de mesure de la saturation en oxygène (SpO₂) du patient, par exemple un capteur transcutané,
- des moyens de mesure de la température corporelle du patient, par exemple un capteur de température en contact avec le corps du patient, et/ou
- des moyens de mesure de la pression artérielle (PA), par exemple un brassard de mesure de pression artérielle.

## Patentansprüche

1. Gerät zur Herzüberwachung (1) eines Patienten, insbesondere eines Patienten mit Herz-Kreislauf-Stillstand (ACR) während einer Herz-Lungen-Wiederbelebung (RCP), das enthält:
- Einrichtungen zur Messung der Herztätigkeit (4, 8, 9) des Patienten,
- mindestens eine graphische Benutzerschnittstelle (2),
- CO₂-Gehalt-Messeinrichtungen (3), um Messungen der Konzentration von vom Patienten erzeugtem CO₂ durchzuführen und CO₂-Gehalt-Messsignale an Signalverarbeitungseinrichtungen (5) zu liefern,
- Signalverarbeitungseinrichtungen (5), die konfiguriert sind, die von den CO₂-Gehalt-Messeinrichtungen (3) kommenden CO₂-Gehalt-Messsignale zu verarbeiten, und
- Speichereinrichtungen (15), um mindestens die verarbeiteten CO₂-Werte zu speichern,
**dadurch gekennzeichnet, dass**:
A) die Signalverarbeitungseinrichtungen (5) konfiguriert sind:
i) einen maximalen CO₂-Gehalt-Wert (Vmax) unter einer Vielzahl von CO₂-Gehalt-Werten auszuwählen, die während eines gegebenen Zeitraums (dt) gemessen und in den Speichereinrichtungen (15) gespeichert werden,
ii) den Schritt i) zu wiederholen, um mehrere aufeinanderfolgende maximale CO₂-Gehalt-Werte (Vmax) zu erhalten, die in einem Zeitfenster (Ft) gemessen werden, das mehrere aufeinanderfolgende Zeiträume (dt) enthält,
iii) mindestens einen CO₂-Gehalt-Mittelwert (Vmoy) ausgehend von den im Zeitfenster (Ft) erhaltenen maximalen CO₂-Gehalt-Werten (Vmax) zu berechnen,
iv) die Schritte i) bis iii) zu wiederholen, um mehrere aufeinanderfolgende CO₂-Gehalt-Mittelwerte (Vmoy) zu erhalten, die ausgehend von in mehreren aufeinanderfolgenden Zeitfenstern (Ft) erhaltenen maximalen CO₂-Gehalt-Werten (Vmax) berechnet werden, und
v) die CO₂-Gehalt-Mittelwerte (Vmoy) an die graphische Benutzerschnittstelle (2) zu übertragen, und
B) die graphische Benutzerschnittstelle (2) konfiguriert ist, die CO₂-Gehalt-Mittelwerte (Vmoy) in Form mindestens einer graphischen Darstellung anzuzeigen.

2. Gerät nach Anspruch 1, **dadurch gekennzeichnet, dass** die aufeinanderfolgenden Zeitfenster (Ft) ein gleitendes Zeitfenster (Ft) sind.

3. Gerät nach einem der Ansprüche 1 oder 2, **dadurch gekennzeichnet, dass** die graphische Benutzerschnittstelle (2) konfiguriert ist, mindestens einen Teil der berechneten aufeinanderfolgenden CO₂-Gehalt-Mittelwerte (Vmoy) anzuzeigen in Form:
- einer Kurve, die von einer Folge graphischer Symbole gebildet wird, wobei jedes graphische Symbol einem CO₂-Gehalt-Mittelwert (Vmoy) entspricht, oder
- eines Balkendiagramms, das mehrere Balken enthält, wobei jeder Balken des Balkendiagramms einem CO₂-Gehalt-Mittelwert (Vmoy) entspricht.

4. Gerät nach einem der Ansprüche 1 oder 2, **dadurch gekennzeichnet, dass**:
- der Zeitraum (dt) zwischen 2 und 10 Sekunden liegt, typischerweise in der Größenordnung von 3 bis 6 Sekunden, und/oder
- das Zeitfenster (Ft) zwischen 20 Sekunden und 10 Minuten liegt, vorzugsweise zwischen 30 Sekunden und 5 Minuten.

5. Gerät nach einem der vorhergehenden Ansprüche, **dadurch gekennzeichnet, dass** die Speichereinrichtungen (15) so konfiguriert sind, dass die gespeicherten CO₂-Gehalt-Werte gelöscht werden:
- entweder nach einer vorab festgelegten Dauer ungleich Null, die zum Beispiel zwischen 2 und 10 Sekunden liegt,
- oder, wenn die Verarbeitungseinrichtungen einen CO₂-Wert Null unter der Vielzahl gespeicherter CO₂-Werte erfassen, insbesondere einen oder mehrere CO₂-Werte Null während einer Dauer zwischen 500 ms und 2 sec.

6. Gerät nach einem der vorhergehenden Ansprüche, **dadurch gekennzeichnet, dass** mehrere aufeinanderfolgende maximale CO₂-Gehalt-Werte (Vmax) in aufeinanderfolgenden Zeiträumen (dt) bestimmt werden, die um eine Pausendauer (dp) zwischen 200 ms und 3 Sekunden voneinander beabstandet sind.

7. Gerät nach einem der vorhergehenden Ansprüche, **dadurch gekennzeichnet, dass** die GUI einen digitalen Bildschirm (12) enthält.

8. Gerät nach einem der vorhergehenden Ansprüche, **dadurch gekennzeichnet, dass** die graphische Anzeige der die Änderungen des mittleren CO₂-Gehalts (Vmoy) darstellenden Trendkurve auf der GUI (2) nach einem regelmäßigen und zyklischen Zeitintervall aufgefrischt wird, vorzugsweise nach einigen Sekunden.

9. Gerät nach einem der vorhergehenden Ansprüche, **dadurch gekennzeichnet, dass** die GUI (2) konfiguriert ist, eine Trendkurve anzuzeigen, die von einer Folge von graphischen Symbolen gebildet wird, die je einem CO₂-Gehalt-Mittelwert (Vmoy) in einem gleitenden Zeitfenster (Ft) zwischen 30 Sekunden und 5 Minuten entsprechen, typischerweise zwischen 1 und 3 Minuten.

10. Gerät nach einem der vorhergehenden Ansprüche, **dadurch gekennzeichnet, dass** es Alarmeinrichtungen enthält, die konfiguriert sind, ausgelöst zu werden, wenn der maximale (Vmax) oder mittlere CO₂-Gehalt-Wert (Vmoy) einen Schwellwert überschreitet, der einer spontanen Wiederaufnahme der Herztätigkeit (RACS) des Patienten entspricht.

11. Gerät nach einem der vorhergehenden Ansprüche, **dadurch gekennzeichnet, dass**:
- die Signalverarbeitungseinrichtungen (5) außerdem konfiguriert sind, die von den Messeinrichtungen der Herztätigkeit (4, 8, 9) des Patienten kommenden Herzsignale zu verarbeiten, und
- die graphische Benutzerschnittstelle (2) konfiguriert ist, die den verarbeiteten Herzsignalen entsprechende Herztätigkeit des Patienten in Form mindestens eines digitalen Werts oder einer graphischen Darstellung anzuzeigen, insbesondere einer Kurve.

12. Gerät nach einem der vorhergehenden Ansprüche, **dadurch gekennzeichnet, dass** die Messeinrichtungen der Herztätigkeit (4, 8, 9) des Patienten konfiguriert sind, die Herzfrequenz, den Herzrhythmus und/oder die elektrische Herztätigkeit (ECG) des Patienten zu messen, vorzugsweise enthalten die Messeinrichtungen der Herztätigkeit (4, 8, 9) des Patienten mehrere Elektroden (8, 9), die auf dem Körper des Patienten platziert werden.

13. Gerät nach einem der vorhergehenden Ansprüche, **dadurch gekennzeichnet, dass** die Messeinrichtungen des CO₂-Gehalts (3) ein Kapnometer enthalten.

14. Gerät nach einem der vorhergehenden Ansprüche, **dadurch gekennzeichnet, dass** es außerdem enthält:
- Einrichtungen zur Messung der Sauerstoffsättigung (SpO₂) des Patienten, zum Beispiel einen transkutanen Sensor,
- Einrichtungen zur Messung der Körpertemperatur des Patienten, zum Beispiel einen Temperaturfühler in Kontakt mit dem Körper des Patienten, und/oder
- Einrichtungen zur Messung des Blutdrucks (PA), zum Beispiel eine Blutdruckmessmanschette.

## Claims

1. Apparatus (1) for cardiac monitoring of a patient, in particular of a patient in cardiorespiratory arrest (CRA) during cardiopulmonary resuscitation (CPR), comprising:
- means (4, 8, 9) for measuring the cardiac activity of the patient,
- at least one graphical user interface (2),
- means (3) for measuring the CO₂ content in order to perform measurements of the concentration of CO₂ produced by said patient and to supply CO₂ content measurement signals to signal-processing means (5),
- signal-processing means (5) configured to process the CO₂ content measurement signals originating from the CO₂ content measurement means (3), and
- storage means (15) for recording at least the CO₂ values processed,
**characterized in that**:
A) the signal-processing means (5) are configured to:
i) select a maximum CO₂ content value (Vmax) from a plurality of CO₂ content values measured during a given time period (dt) and recorded in the storage means (15),
ii) repeat step i) in order to obtain several successive maximum CO₂ content values (Vmax) measured over a time window (Ft) comprising several successive time periods (dt),
iii) calculate at least one mean CO₂ content value (Vmean) from the maximum CO₂ content values (Vmax) obtained over the time window (Ft),
iv) repeat steps i) to iii) in order to obtain several successive mean CO₂ content values (Vmean) calculated from maximum CO₂ content values (Vmax) obtained over several successive time windows (Ft), and
v) transmit the mean CO₂ content values (Vmean) to the graphical user interface (2), and
B) the graphical user interface (2) is configured to display the mean CO₂ content values (Vmean) in the form of at least one graphical representation.

2. Apparatus according to Claim 1, **characterized in that** the successive time windows (Ft) are a sliding time window (Ft).

3. Apparatus according to either of Claims 1 and 2, **characterized in that** the graphical user interface (2) is configured to display at least some of the successive calculated mean CO₂ content values (Vmean) in the form:
- of a curve formed by a succession of graphical symbols, each graphical symbol corresponding to a mean CO₂ content value (Vmean), or
- of a bar graph comprising several bars, each bar of said bar graph corresponding to a mean CO₂ content value (Vmean).

4. Apparatus according to either of Claims 1 and 2, **characterized in that**:
- the time period (dt) is between 2 and 10 seconds, typically of the order of 3 to 6 seconds, and/or
- the time window (Ft) is between 20 seconds and 10 minutes, preferably between 30 seconds and 5 minutes.

5. Apparatus according to one of the preceding claims, **characterized in that** the storage means (15) are configured such that the recorded CO₂ content values are erased:
- either after a non-zero duration that is prefixed, for example between 2 and 10 seconds,
- or when the processing means detect a zero CO₂ value among the stored plurality of CO₂ values, in particular one or more zero CO₂ values for a period of between 500 ms and 2 seconds.

6. Apparatus according to one of the preceding claims, **characterized in that** several successive maximum CO₂ content values (Vmax) are determined over successive time periods (dt), spaced apart from one another by a pause time (dp) of between 200 ms and 3 seconds.

7. Apparatus according to one of the preceding claims, **characterized in that** the GUI comprises a digital screen (12).

8. Apparatus according to one of the preceding claims, **characterized in that** the graphical display, on the GUI (2), of the trend curve representing the variations in the mean CO₂ content (Vmean) is refreshed after a regular and cyclical time interval, preferably after a few seconds.

9. Apparatus according to one of the preceding claims, **characterized in that** the GUI (2) is configured to display a trend curve formed by a succession of graphical symbols each corresponding to a mean CO₂ content value (Vmean) over a sliding time window (Ft) of between 30 seconds and 5 minutes, typically of 1 to 3 minutes.

10. Apparatus according to one of the preceding claims, **characterized in that** it comprises alarm means configured to trigger when the maximum (Vmax) or mean (Vmean) CO₂ content value exceeds a threshold value corresponding to an RSCA of the patient.

11. Apparatus according to one of the preceding claims, **characterized in that**:
- the signal-processing means (5) are additionally configured to process the cardiac signals originating from the means (4, 8, 9) for measuring the cardiac activity of the patient, and
- the graphical user interface (2) is configured to display the cardiac activity of the patient corresponding to the processed cardiac signals, in the form of at least one numerical value or a graphical representation, in particular a curve.

12. Apparatus according to one of the preceding claims, **characterized in that** the means (4, 8, 9) for measuring the cardiac activity of the patient are configured to measure the heart rate, the cardiac rhythm and/or the electrical cardiac activity (ECG) of the patient, and preferably the means (4, 8, 9) for measuring the cardiac activity of the patient comprise several electrodes (8, 9) which are placed on the patient's body.

13. Apparatus according to one of the preceding claims, **characterized in that** the means (3) for measuring the CO₂ content comprise a capnometer.

14. Apparatus according to one of the preceding claims, **characterized in that** it further comprises:
- means for measuring the patient's oxygen saturation (SpO₂), for example a transcutaneous sensor,
- means for measuring the patient's body temperature, for example a temperature sensor in contact with the patient's body, and/or
- means for measuring the arterial pressure (AP), for example a cuff for measuring arterial pressure.
